**Europäisches Patentamt**

**European Patent Office**

**Office européen des brevets**

(19)

(11) Veröffentlichungsnummer : **0 031 886**
**B1**

(12) **EUROPÄISCHE PATENTSCHRIFT**

(45) Veröffentlichungstag der Patentschrift :
**11.04.84**

(21) Anmeldenummer : **80107045.9**

(22) Anmeldetag : **14.11.80**

(51) Int. Cl.³ : **C 07 C 53/124**, C 07 C 51/14,
C 07 C 69/24, C 07 C 67/38,
C 07 C 51/353

(54) **Verfahren zur Herstellung von Isobuttersäure oder ihrer niederen Alkylester.**

(30) Priorität : **20.12.79 DE 2951289**
**06.09.80 DE 3033655**

(43) Veröffentlichungstag der Anmeldung :
**15.07.81 Patentblatt 81/28**

(45) Bekanntmachung des Hinweises auf die Patenterteilung : **11.04.84 Patentblatt 84/15**

(84) Benannte Vertragsstaaten :
**DE FR GB IT NL**

(56) Entgegenhaltungen :
**US-A- 2 975 199**
**US-A- 3 005 846**
**US-A- 3 052 698**
**US-A- 3 661 951**
**Takezaki et al., Bull. Jap. Petrol. Inst. 8(1966), 31-38**
**Die Akte enthält technische Angaben, die nach dem Eingang der Anmeldung eingereicht wurden und die nicht in dieser Patentschrift enthalten sind.**

(73) Patentinhaber : **Röhm GmbH**
**Kirschenallee Postfach 4242**
**D-6100 Darmstadt 1 (DE)**

(72) Erfinder : **Besecke, Siegmund, Dr. Dipl.-Chem.**
**Heidelberger Landstrasse 62**
**D-6100 Darmstadt 13 (DE)** .
Erfinder : **Schröder, Günter, Dr.**
**Leipziger Strasse 7**
**D-6100 Ober-Ramstadt (DE)**
Erfinder : **Siegert, Hermann-Josef, Dr. Dipl.-Chem.**
**Inselstrasse 21**
**D-6100 Darmstadt (DE)**
Erfinder : **Gänzler, Wolfgang, Dr.**
**Wixhäuser Strasse 11**
**D-6100 Darmstadt-Arheilgen (DE)**

# 0 031 886

Verfahren zur Herstellung von Isobuttersäure oder ihrer niederen Alkylester

Die Erfindung betrifft ein verbessertes Verfahren zur Herstellung von Isobuttersäure oder ihrer niederen Alkylester. Diese Verbindungen können durch Dehydrierung in Methacrylsäure oder deren niedere Alkylester übergeführt werden, die wiederum Ausgangsstoffe für wertvolle Kunststoffe sind.

Die Herstellung von Isobuttersäure durch die Koch'sche Synthese, ausgehend von Propylen, ist aus der DE-PS 942 987 bekannt. Unter Mitverwendung von Wasser gelangt man zur Isobuttersäure, während bei Mitverwendung von niederen Alkanolen, insbesondere Methanol, die entsprechenden niederen Alkylester entstehen. Man kann auch von Isopropylalkohol ausgehen und kommt dann ohne Mitverwendung von Wasser zur Isobuttersäure. Als Koch'scher Katalysator für diese Umsetzungen ist unter anderem Fluorwasserstoff eingesetzt worden.

Die Herstellung von Isobuttersäure oder ihrer Ester durch Koch'sche Synthese ist bisher nicht im technischen Maßstab verwirklicht worden. Obwohl eingehende Untersuchungen der Reaktion vorliegen und zahlreiche Verfahrensvarianten und Versuchsbedingungen geprüft worden sind, wurden die für ein technisches Verfahren erforderliche hohe Selektivität und Ausbeute bei einer tragbaren Verweilzeit bzw. Raum-Zeit-Ausbeute nicht erreicht.

Nach der US-PS 3 127 438 wird Propylen in einem zweistufigen Verfahren in Isobuttersäure übergeführt. In der ersten Verfahrensstufe werden Propylen und Kohlenmonoxid in wasserfreiem Fluorwasserstoff umgesetzt. Das Zwischenprodukt wird in der zweiten Reaktionsstufe mit einem Alkohol zum Ester umgesetzt. Während die erste Reaktionsstufe meistens in 15 bis 20 min abgeschlossen ist, benötigt die zweite Reaktionsstufe im allgemeinen eine längere Reaktionszeit. Die gesamte Verfahrensdauer übersteigt in der Regel eine Stunde. Bei einer Gesamtdauer von 30 min wurde nur etwa die Hälfte des eingesetzten Propylens in den Isobuttersäureester umgewandelt.

Wesentlich höhere Ausbeuten an Carbonsäuren werden bei dem Verfahren gemäß US-PS 3 661 951 in einem einstufigen Verfahren erhalten, bei dem man ein etwa äquimolares Gemisch aus einem Olefin und Wasser mit Kohlenmonoxid in einem großen Überschuß des als Katalysator dienenden Fluorwasserstoffs umsetzt. Um Ausbeuten in der Größenordnung von 90 % zu erreichen, werden Reaktionszeiten von mehr als 30 min, meistens mehr als 60 min bei Temperaturen von 23 bis 38 °C benötigt.

Beim Verfahren der US-PS 3 005 846 wird ein Verfahren in Gegenwart ähnlich großer Mengen Fluorwasserstoff, der 5-30 % Wasser oder, wenn die Herstellung des entsprechenden Carbonsäureesters beabsichtigt ist, 2 bis 15 Mol.-% Alkohol enthält, bei einer Temperatur zwischen − 12 und 93 °C umgesetzt, wobei die Temperatur umso höher liegen soll, je höher der Wassergehalt im Reaktionsgemisch ist. Wenn auch für die Dauer Umsetzung ein allgemeiner Spielraum von 1 Minute bis zu 1 Stunde angegeben wird, so wird bei der praktischen Durchführung des Verfahrens in der Regel mehr als 1 Stunde benötigt.

Y. Takezaki et al. (Bull. Jap. Petrol. Inst., Band 8, 1966, S. 31-38) haben die Koch'sche Synthese von Isobuttersäure aus Propylen, Wasser und Kohlenmonoxid in Fluorwasserstoff systematisch untersucht und ein Optimum für hohe Ausbeute und hohe Umsetzungsgeschwindigkeit ermittelt. Gemäß diesem Optimum setzt man je Mol Propylen 15 Mol Fluorwasserstoff ein, der mit 20 Gew.-% Wasser vermischt ist, und führt die Umsetzung bei 94 °C unter einem Gesamtdruck von 190 bar oder mehr durch. Unter diesen Bedingungen wurde in 20 min eine Ausbeute von 70 % d. Th. an Isobuttersäure erhalten. Bei höherem oder niedrigerem Wassergehalt nimmt die Ausbeute ab, ebenso bei höherer oder niedrigerer Umsetzungstemperatur. Mit steigender Temperatur nimmt die Bildung unerwünschter Oligomerisierungsprodukte des Propylens zu. Bei Drucken von 50 bis 100 bar wurden lediglich Ausbeuten von etwa 50 % erreicht.

Gemäß GB-PS 1 167 116 werden Olefine mit 4 oder mehr Kohlenstoffatomen in Gegenwart von Fluorwasserstoff mit Kohlenmonoxid und überschüssigem Wasser bei 20-90 °C umgesetzt. Propylen ist als Ausgangsstoff ausgeschlossen. Bei diesem Verfahren werden insbesondere in kontinuierlicher Betriebsweise hohe Ausbeuten und Selektivitäten an den entsprechenden Carbonsäuren erhalten, wenn ein gesättigter Kohlenwasserstoff als Verdünnungsmittel für das Olefin mitverwendet wird. Die erforderliche Verweilzeit beträgt 40 bis 60 Minuten.

Aus der DE-OS 20 26 031 (Beispiel 11) ist es bekannt, Isopropylformiat unter hohem Druck und bei hoher Temperatur mit Kobaltbromid als Katalysator in N-Methylpyrrolidon zu isomerisieren. Im Gegensatz zur Koch'schen Synthese wird bei diesem Verfahren ein Gemisch von Iso- und n-Buttersäure erhalten, das destillativ schwierig aufzutrennen ist. Die Gesamtausbeute an beiden Säuren beträgt 76 %.

Die langen Reaktionszeiten bei den bekannten diskontinuierlichen Verfahren und die ebenfalls langen Verweilzeiten bei dem erwähnten kontinuierlichen Verfahren ließen die kontinuierliche Durchführung der Koch'schen Synthese von Isobuttersäure aus Propylen, Kohlenmonoxid und Wasser im großtechnischen Maßstab wenig aussichtsreich erscheinen, zumal dann kurze Verweilzeiten und hohe Ausbeuten und Selektivitäten unerläßlich sind. Gegen eine Beschleunigung der Umsetzungsgeschwindigkeit durch Erhöhen der Temperatur über 94 °C hinaus sprach die von Takezaki (loc. cit) angegebene Erfahrung, daß die Oligomerisierung des Propylens mit steigender Temperatur erheblich zunimmt.

In der Tat erweist sich die Oligomerenbildung als das am schwersten überwindbare Problem bei der Koch'schen Synthese mit Propylen. Die Tatsache, daß die GB-PS 1 167 116 auf Olefine mit mehr als 3 Kohlenstoffatomen beschränkt ist und in der US-PS 3 005 846 die Umsetzung von Propylen zwar beansprucht, aber in keinem Ausführungsbeispiel beschrieben ist, findet in dieser Schwierigkeit ihre Erklärung. Selbst höhere Olefine ergeben erhebliche und oft sogar überwiegende Anteile an Oligomerisierungsprodukten, wie aus den Beispielen der letztgenannten Patentschrift hervorgeht. Selbst Ausbeuteverluste von 8 bis 10 % in Form von unverwertbaren Oligomerisierungsprodukten, die bei allen bekannten Verfahren der Koch'schen Synthese mit Propylen anfallen, sind für die Erzeugung eines Grundstoffes für die Kunststoffherstellung untragbar.

Der Erfindung liegt die Aufgabe zugrunde, diese Synthese unter Bedingungen durchzuführen, die ein großtechnisches Verfahren zur Herstellung von Isobuttersäure oder ihren Estern aus Propylen, Kohlenmonoxid und Wasser bzw. einem Alkohol zulassen. Das schließt die Forderung nach kontinuierlicher Verfahrensführung, hoher Ausbeute und Selektivität und damit weitgehender Verminderung des Oligomerenproblems, sowie nach hoher Raumzeitausbeute, gleichbedeutend mit einer kurzen Verweilzeit, ein.

Der Lösung der gestellten Aufgabe ging die Erkenntnis voraus, daß hohe, der Oligomerenbildung an sich förderliche Temperaturen von mindestens 80, insbesondere über 100 °C, im Interesse einer hohen Umsetzungsgeschwindigkeit unverzichtbar sind. Es wurde gefunden, daß selbst unter diesen Bedingungen die Oligomerenbildung erheblich eingeschränkt werden kann, wenn die Propylenkonzentration im reagierenden Gemisch dauernd auf einem niedrigen Niveau gehalten wird. Die Verwirklichung dieses Prinzips führte zu dem Verfahren der Erfindung, bei dem Isobuttersäure oder ihre niederen Alkylester durch Umsetzung von Propylen, Kohlenmonoxid und Wasser bzw. einem niederen Alkohol oder durch Umsetzung von Additionsverbindungen aus zwei der am Reaktionsgemisch beteiligten Stoffe in Gegenwart von Fluorwasserstoff als Koch'schem Katalysator kontinuierlich in einer Stufe unter hoher Rückvermischung des reagierenden Gemisches in einer Verweilzeit der flüssigen Phase unter 20 Minuten bei einer Temperatur zwischen 80 und 160 °C hergestellt werden. Erfindungsgemäß wird in dem reagierenden Gemisch der Gehalt an Propylen unterhalb 1 Gew.-% (bezogen auf das Gesamtgewicht des aus der flüssigen und der gasförmigen Phase bestehenden Reaktionsgemisches) und der Gehalt an Wasser bzw. dem niederen Alkohol unter 5 Mol.-% (bezogen auf Fluorwasserstoff) gehalten. Alternativ kann Isopropylformiat eingesetzt werden, wodurch die getrennte Zuführung von Propylen und Wasser entfällt. Die kontinuierliche Verfahrensweise bietet die Bedingung für die Zufuhr des Propylens nach Maßgabe des laufenden Umsatzes. Die hohe Rückvermischung des reagierenden Gemisches ist notwendig, um jeden örtlichen Konzentrationsanstieg an der Einführungsstelle des Propylens zu unterbinden.

Die Propylenkonzentration ist bei der jeweils herrschenden Umsetzungsgeschwindigkeit durch die Zuführungsgeschwindigkeit der Ausgangsstoffe und damit praktisch durch die Verweilzeit einstellbar. Die Verweilzeit muß jeweils für die fast vollständige Umsetzung des zugeführten Propylens ausreichen. Unter der Propylenkonzentration wird der prozentuale Gewichtsanteil des Propylens am Gesamtgewicht des aus der flüssigen und gasförmigen Phase bestehenden Reaktionsgemisches verstanden.

Es war nicht voraussehbar, daß sich das Problem der Oligomerenbildung durch die beanspruchte kontinuierliche Betriebsweise bei Propylenkonzentration unter 1 %, vorzugsweise unter 0,7 %, in befriedigender Weise lösen lassen würde.

Als wesentlich für eine hohe Umsetzungsgeschwindigkeit erwies es sich, den Gehalt an Wasser bzw. dem zur Esterbildung eingesetzten niederen Alkohol im Reaktionsgemisch unter 5 Mol.-%, bezogen auf Fluorwasserstoff, insbesondere unter 2 Mol.-% zu halten, was durch die zugeführte Wasser- bzw. Alkoholmenge steuerbar ist. Dadurch wird die Oligomerenbildung weiter unterdrückt und gleichzeitig die Umsetzungsgeschwindigkeit erhöht, was wiederum kürzere Verweilzeiten gestattet. Das Molverhältnis von Propylen zu Wasser bzw. Alkohol soll vorzugsweise etwa äquivalent sein, kann aber vom Verhältnis 1 : bis zu Werten zwischen 0,8 : 1 und 2 : 1 abweichen, um den Wasser- bzw. Alkoholgehalt im Reaktionsgemisch in der gewünschten Höhe zu halten. Mit Vorteil liegt das Verhältnis Propylen/Wasser bzw. niederem Alkohol bei 1 : (0,9-1,1), insbesondere bei 1 : (0,95 bis 1,0). Fluorwasserstoff wird bevorzugt in einer Menge von 5 bis 15 Mol, insbesondere 8 bis 12 Mol, pro Mol Propylen oder Isopropylalkohol angewandt.

Unter optimalen Bedingungen läßt das Verfahren der Erfindung eine Gesamtausbeute an Isobuttersäureverbindungen von mehr als 90 % d. Th., davon mehr als 80 % d. Th. in Form von freier Isobuttersäure bei Verweilzeiten von wenigen Minuten erreichen und eröffnet damit erstmals die Möglichkeit zu einer großtechnischen Isobuttersäure-Synthese.

Wenn die unmittelbare Bildung eines Esters der Isobuttersäure angestrebt wird, so wird anstelle von Wasser der entsprechende Alkohol eingesetzt. Es kommen vor allem die niederen primären Alkanole mit 1 bis 4 C-Atomen in Betracht. Methanol ist das bevorzugte Alkanol. Für die Umwandlung der Isobuttersäureverbindungen in Methacrylverbindungen bietet die freie Isobuttersäure Vorteile vor ihren Estern, so daß die Herstellung der freien Säure gegenüber der Herstellung der Isobuttersäureester die bevorzugte Ausführungsform der Erfindung ist.

Die bevorzugten Ausgangsstoffe für das Verfahren der Erfindung sind Propylen, Kohlenmonoxid und Wasser bzw. der niedere Alkohol. Soweit sich aus Paaren der am Reaktionsgemisch beteiligten Stoffe binäre Additionsverbindungen bilden lassen, können diese anstatt oder neben den Bildungskomponenten eingesetzt werden. Propylen kann mit 1 Mol Wasser Isopropylalkohol und mit 1/2 Mol Wasser Diisopropyläther bilden. Mit Fluorwasserstoff bildet sich leicht Isopropylfluorid. Kohlenmonoxid kann mit Wasser zu Ameisensäure, mit dem niederen Alkohol zu dem entsprechenden Ameisensäureester und mit Fluorwasserstoff zu Formylfluorid umgesetzt werden. Aus dem niederen Alkohol und Propylen kann der entsprechende Isopropylalkyläther entstehen. Diese binären Additionsprodukte reagieren beim Verfahren der Erfindung meistens ebenso leicht wie die Bildungskomponenten zu Isobuttersäure oder ihrem Ester. Da sie in einer besonderen Verfahrensstufe hergestellt werden müssen, empfiehlt sich ihre Verwendung anstelle oder neben den Bildungskomponenten nur in Ausnahmefällen. Unter den erwähnten Additionsprodukten nimmt Isopropylalkohol wegen seiner leichten Herstellbarkeit eine bevorzugte Stellung ein. Man erhält ihn z. B. aus einem Gemisch aus Propylen und Wasser an einem sauren Ionenaustauscher. Es gibt auch ein ternären Additionsprodukt aus drei der beteiligten Stoffe, nämlich Isopropylformiat, das aus Propylen, Kohlenmonoxid und Wasser zusammengesetzt ist. Isopropylformiat nimmt als Ausgangsmaterial für das Verfahren der Erfindung insofern eine Sonderstellung ein, als sein Einsatz im Verfahren der Erfindung nicht streng an die engen Verfahrensbedingungen gebunden ist, die für das Gemisch der Bildungskomponenten oder für die binären Additionsverbindungen gelten.

Die Umsetzung von Isopropylformiat zu Isobuttersäure ist eine reine Isomerisierung, so daß keine weiteren Ausgangsstoffe erforderlich sind. Es wird allerdings bevorzugt, die Umsetzung in Gegenwart von Kohlenmonoxid durchzuführen, das bei der Umsetzung nicht verbraucht und aus dem Reaktionsgemisch vollständig zurückgewonnen wird. Die Umsetzungstemperatur liegt im allgemeinen zwischen 20 und 150, vorzugsweise zwischen 60 und 120 °C. Die Verweilzeit im Reaktionsgemäß kann zwischen 1 und 200 min liegen. Fluorwasserstoff wird in einer Menge von 5 bis 20 Säureäquivalenten je Mol Isopropylformiat eingesetzt.

Die bei der Koch'schen Synthese in anderen Fällen häufig auftretenden Oligomerisierungen bleiben auch bei der Verfahrensform mit Isopropylformiat weitgehend aus, so daß eine nahe an 100 % reichende Selektivität für Isobuttersäure erreichbar ist.

Weiterhin wird eine praktisch quantitative Umsetzung erreicht.

Da die Additionsprodukte im Reaktionsgemisch im allgemeinen schnell in ihre Bildungskomponenten zerfallen, ist bei der Bemessung der Zuführungsgeschwindigkeit bzw. der Verweilzeit der beanspruchte Grenzwert der Propylenkonzentration und gegebenenfalls auch der Wasser- bzw. Alkoholkonzentration in entsprechender Weise zu beachten.

Der Gesamtdruck im Reaktior setzt sich aus den Partialdrucken von Propylen, Kohlenmonoxid, Fluorwasserstoff und dem Dampfdruck der in der flüssigen Phase vorliegenden organischen Substanzen zusammen. Der Gesamtdruck liegt unter Reaktionsbedingungen vorzugsweise im Bereich von 50 bis 150 bar, insbesondere 80 bis 140 bar. Der CO-Partialdruck beträgt mindestens 35 und vorzugsweise 60 bis 130 bar.

Bei der bevorzugten Ausführungsform der Erfindung, die von dem Gemisch aus Propylen, Kohlenmonoxid und Wasser oder Alkohol bzw. den erwähnten binären Additionsverbindungen ausgeht, werden die engen Verfahrensbedingungen, wie im Anspruch 1 angegeben, angewandt, wie im nachfolgenden noch näher erläutert wird.

Das Verfahren kann in einem breiten Temperaturbereich von 80 bis 160 °C durchgeführt werden, wobei die Reaktionsgeschwindigkeit mit der Temperatur zunimmt. Der bevorzugte Temperaturbereich erstreckt sich von 100 bis 140 °C.

In Verweilzeiten von 2 bis 15 min werden bei 100 bis 120 °C Gesamtausbeuten über 90 % d. Th. und Selektivitäten für Isobuttersäure über 93 % erreicht.

Das Verfahren der Erfindung kann in üblichen Druckreaktoren mit hoher Rückvermischung durchgeführt werden, die unter den Verfahrensbedingungen gegen Fluorwasserstoff beständig sein müssen. Als Werkstoffe dafür eignen sich Nickel oder Nickellegierungen, wie z. B. Monel[®], Inconel[®] oder Hastelloy[®]. Die erforderliche Größe des Druckreaktors ergibt sich aus der stündlich durchzusetzenden Menge und der Raum-Zeit-Ausbeute. Da die Kosten von Druckreaktoren, insbesondere solchen aus Speziallegierungen, mit der Größe stark zunehmen, wirken sich die kurzen Verweilzeiten und die damit verbundenen hohen Raum-Zeit-Ausbeuten vorteilhaft auf die Anlagekosten aus. Gegenüber einer Verweilzeit von 1 Stunde, die bei vielen bekannten Verfahren erforderlich ist, gestatten die erfindungsgemäß erreichbaren Verweilzeiten von z. B. 5 bis 10 min eine Verminderung der Reaktorgröße auf ein Sechstel bis ein Zwölftel. Im gleichen Ausmaß vermindert sich das Volumen von Sicherheits- und Entspannungsgefäßen.

Neben einfachen Rührautoklaven kommen vor allem Gas-Flüssig-Reaktoren mit dispergierter Gasphase in Betracht. Dazu gehören Blasensäulen oder Strahlreaktoren, die eine innige Berührung der Flüssigphase mit der Gasphase und eine hohe Umwälzrate des flüssigen Reaktionsgemisches ermöglichen. Die gasförmigen Bestandteile des Reaktionsgemisches, vor allem Propylen und Kohlenmonoxid werden vorzugsweise vor dem Eintritt in den Reaktor gemischt und als Gasgemisch eingeführt. Aus dem oberen Teil des Reaktors kann Gas abgenommen und zusammen mit dem Frischgas im Kreislauf wieder zugemischt werden.

Die flüssige Phase des Reaktors wird kontinuierlich über ein Entspannungsventil abgezogen und in üblicher Weise aufgearbeitet. Vorzugsweise wird das Rohprodukt destillativ aufgearbeitet. Dabei werden in einem ersten Destillationsschritt die niedrig siedenden Bestandteile, vor allem Fluorwasserstoff und eventuell nicht umgesetztes Propylen, bei Normaldruck oder leichtem Überdruck bis z. B. 4 bar abdestilliert, komprimiert und in den Reaktor zurückgeführt. Wenn dazu eine kurze Destillationskolonne verwendet wird, kann der Fluorwasserstoff im wesentlichen vollständig von den organischen Reaktionsprodukten abgetrennt werden.

Zusammen mit dem Fluorwasserstoff werden alle niedrigsiedenden Nebenprodukte verflüchtigt, z. B. Isopropylalkohol, Isopropylfluorid, Isobuttersäurefluorid, Isobuttersäureisopropylester und Diisopropyläther. Sie gelangen zusammen mit dem Fluorwasserstoff in den Reaktor zurück und nehmen an der weiteren Umsetzung zu Isobuttersäure teil. Zum Teil sind sie mit Wasser leicht zersetzbar. Es ist daher von Vorteil, dem Rohprodukt bei der Destillation etwas Wasser zuzuführen und dadurch die Hydrolyse von Isobuttersäurefluorid bzw. Isobuttersäureisopropylester zu Isobuttersäure zu fördern. Die Coprodukte der Hydrolyse, nämlich Fluorwasserstoff und Isopropylalkohol, gelangen in den Reaktor zurück.

Der beim ersten Destillationsschritt flüssig zurückgebliebene Teil des Rohproduktes besteht hauptsächlich aus Isobuttersäure und in geringen Mengen aus Hochsiedern, Oligomeren des Propylens u. dergl. In einem zweiten Destillationsschritt, vorzugsweise in einer weiteren kurzen Kolonne, wird die Isobuttersäure verdampft und entweder in an sich bekannter Weise kondensiert oder — bevorzugt — unmittelbar in Dampfform einem Reaktor zur oxidativen Dehydrierung zu Methacrylsäure zugeführt. Da diese Reaktion bzw. die entsprechenden Anlagenteile von Fluorwasserstoff gestört werden können, schickt man die dampfförmige Isobuttersäure mit Vorteil über eine geeignete Absorptionssäule. Zur restlosen Entfernung von Fluorwasserstoff eignet sich beispielsweise eine Bauxitfüllung. Die hochsiedenden oder nichtflüchtigen Destillationsrückstände werden verworfen oder verbrannt.

Beispiel 1

Kontinuierliche Umsetzung von Propylen, CO und Wasser

Die Umsetzung wird in einem Rührautoklaven aus einer Nickellegierung (Hastalloy C4ʳ) von 60 ml freiem Reaktorvolumen mit Begasungsrührer durchgeführt. Die Rührgeschwindigkeit beträgt 1 000 $U_p$. Der Autoklav ist mit einer durch die Rührer führenden Gaszuführungsleitung sowie weiteren Zu- und Ableitungen ausgerüstet und kann mittels einer elektrischen Heizung erwärmt werden.

CO wird zum Teil über den Begasungsrührer eingeleitet. Die übrigen Komponenten werden über getrennte Leitungen eingepumpt.

Propylen, Wasser, Fluorwasserstoff und CO werden bei Reaktortemperaturen von 100 und 120 °C und einem Betriebsdruck von 120 bar im Molverhältnis 1 : 0,96 : 10 : 1,5 umgesetzt. Die Verweilzeit wird durch Veränderung der Durchsatzmengen zwischen 4 und 15 min variiert. Im stationären Zustand werden folgende Ausbeuten und Selektivitäten erreicht :

| Verweilzeit [min] | Reaktionstemp. 100° | | Reaktionstemp. 120° | |
|---|---|---|---|---|
| | Ausbeute IBS* [%] | Selektivität [%] | Ausbeute IBS* [%] | Selektivität [%] |
| 4 | 81 | 86 | 91 | 95 |
| 7,5 | 82 | 92 | 93 | 96 |
| 15 | 93 | 96 | 94 | 96 |

*IBS = Isobuttersäure und zu Isobuttersäure hydrolysierbare Derivate, insbesondere Isopropylester.

Bei einer Verweilzeit von 3 min wurde das Propylen-Wasser-Verhältnis zwischen 1 : 0,96 und 1 : 0,99 variiert. Bei 120 °C wurden folgende Ergebnisse erhalten :

| Propylen/Wasser [Mol] | Ausbeute IBS [%] | Selektivität [%] |
|---|---|---|
| 1 : 0,96 | 85 | 91 |
| 1 : 0,97 | 89 | 93 |
| 1 : 0,98 | 92 | 95 |
| 1 : 0,99 | 89 | 93 |

Beispiel 2

Kontinuierliche Umsetzung von Isopropylformiat und CO

In der in Beispiel 1 beschriebenen Apparatur wird kontinuierlich ein Reaktionsgemisch aus Isopropylformiat, technischem Fluorwasserstoff und Kohlenmonoxid im Molverhältnis 1 : 10 : 0,5 bei 70 °C und bei 120 °C umgesetzt.

Ergebnisse :

| | | |
|---|---|---|
| Reaktionstemperatur | 70 °C | 120 °C |
| Verweilzeit | 30 min | 5 min |
| Druck im Reaktor | 100 bar | 110 bar |
| Ausbeute an Isobuttersäure | 97 % | 98 % |
| Selektivität | > 98 % | > 98 % |

**Ansprüche**

1. Verfahren zur Herstellung von Isobuttersäure oder ihren niederen Alkylestern durch Koch'sche Synthese durch Umsetzung von Propylen, Kohlenmonoxid und Wasser bzw. einem niederen Alkohol, wobei die genannten Ausgangsstoffe ganz oder teilweise durch die entsprechenden Additionsverbindungen der am Reaktionsgemisch beteiligten Stoffe ersetzt sein können, in Gegenwart von Fluorwasserstoff als Koch'schem Katalysator in einer Stufe, dadurch gekennzeichnet, daß die genannten Ausgangsstoffe, die ganz oder teilweise durch die entsprechenden Additionsverbindungen aus zwei der am Reaktionsgemisch beteiligten Stoffe ersetzt sein können, kontinuierlich mit einer solchen Zuführungsgeschwindigkeit in das Reaktionsgemisch eingeleitet und mit diesem so durchmischt werden, daß bei einer Umsetzungstemperatur zwischen 80 und 160 °C in dem reagierenden Gemisch ein Gehalt an Propylen von 1 Gew.-% (bezogen auf das Gesamtgewicht des aus der flüssigen und der gasförmigen Phase bestehenden Reaktionsgemisches) und ein Gehalt an Wasser oder dem niederen Alkohol von 5 Mol.-% (bezogen auf Fluorwasserstoff) nicht erreicht und nicht durch einen örtlichen Konzentrationsanstieg an der Einleitungsstelle überschritten werden und die Verweilzeit der flüssigen Phase unter 20 Minuten liegt.

2. Verfahren nach Anspruch 2, dadurch gekennzeichnet, daß der Gehalt an Wasser oder dem niederen Alkohol dauernd unter 2 Mol.-% gehalten wird.

3. Verfahren nach den Ansprüchen 1 bis 3, dadurch gekennzeichnet, daß es bei einem Gesamtdruck von 50 bis 150 bar durchgeführt wird.

4. Verfahren nach den Ansprüchen 1 bis 4, dadurch gekennzeichnet, daß es bei einer Temperatur zwischen 100 und 140 °C durchgeführt wird.

5. Verfahren nach den Ansprüchen 1 bis 5, dadurch gekennzeichnet, daß es mit einer Verweilzeit der flüssigen Phase von 2 bis 10 min durchgeführt wird.

6. Verfahren nach den Ansprüchen 1 bis 6, dadurch gekennzeichnet, daß es in Gegenwart von 5 bis 15 Mol HF/Mol Propylen durchgeführt wird.

7. Verfahren nach den Ansprüchen 1 bis 7, dadurch gekennzeichnet, daß als niederer Alkohol Methanol eingesetzt wird.

8. Verfahren nach den Ansprüchen 1 bis 8, dadurch gekennzeichnet, daß das Wasser bzw. der niedere Alkohol und Propylen kontinuierlich in einem Molverhältnis von (0,9 bis 1,1) : 1 zugeführt werden.

9. Verfahren nach den Ansprüchen 1 bis 9, dadurch gekennzeichnet, daß Kohlenmonoxid und Propylen gasförmig gemischt und in Form dieses Gemisches im Fluorwasserstoff umgesetzt werden.

10. Verfahren nach den Ansprüchen 1 bis 10, dadurch gekennzeichnet, daß es in einem Gas-Flüssig-Reaktor mit dispergierter Gasphase durchgeführt wird.

**Claims**

1. Process for the preparation of isobutyric acid or the lower alkyl esters thereof by Koch synthesis, by reacting propylene, carbon monoxide and water or a lower alcohol, wherein the starting materials specified may be wholly or partially replaced by the corresponding addition compounds of the substances included in the reaction mixture, in the presence of hydrogen fluoride as the Koch catalyst, in a single step, characterised in that the starting materials specified, which may be wholly or partially replaced by the corresponding addition compounds of two of the substances included in the reaction mixture, are continuously introduced into the reaction mixture at such a rate and mixed with the mixture in such a way that, at a reaction temperature of between 80 and 160 °C, a propylene content of 1 % by weight (based on the total weight of the reaction mixture consisting of a liquid phase and a gaseous phase) and a water or lower alcohol content of 5 mol.-% (based on hydrogen fluoride) in the reacting mixture are not attained and cannot be exceeded as a result of any local increase in concentration at the point of introduction, and the retention time of the liquid phase is less than 20 minutes.

2. Process as claimed in claim 1, characterised in that the content of water or lower alcohol is kept permanently below 2 mol.-%.

3. Process as claimed in claims 1 to 2, characterised in that it is carried out under a total pressure of from 50 to 150 bar.

4. Process as claimed in claims 1 to 3, characterised in that it is carried out at a temperature of between 100 and 140 °C.

5. Process as claimed in claims 1 to 4, characterised in that it is carried out with a retention time for the liquid phase of from 2 to 10 minutes.

6. Process as claimed in claims 1 to 5, characterised in that it is carried out in the presence of from 5 to 15 mol of HF per mol of propylene.

7. Process as claimed in claims 1 to 6, characterised in that methanol is used as the lower alcohol.

8. Process as claimed in claims 1 to 7, characterised in that water or the lower alcohol and propylene are fed in continuously in a molar ratio of (0.9 to 1.1) : 1.

9. Process as claimed in claims 1 to 8, characterised in that carbon monoxide and propylene are mixed together in gaseous form and are reacted in the form of this mixture in the hydrogen fluoride.

10. Process as claimed in claims 1 to 9, characterised in that it is carried out in a gas-liquid reactor with a dispersed gas phase.

**Revendications**

1. Procédé de préparation d'acide isobutyrique ou de ses esters alkyliques inférieurs par synthèse de Koch par la mise en réaction de propylène, d'oxyde de carbone et d'eau ou d'un alcool inférieur, ces substances de départ pouvant être remplacées en totalité ou en partie par les composés d'addition correspondants des substances entrant dans la composition du mélange réactionnel, en présence de fluorure d'hydrogène servant de catalyseur de Koch et en un seul temps, caractérisé en ce que les substances de départ mentionnées, qui peuvent être remplacées en totalité ou en partie par les composés d'addition correspondante de deux des substances entrant dans la composition du mélange réactionnel, sont introduites en continu dans le mélange réactionnel et y sont mélangées intimement avec une vitesse d'arrivée telle qu'avec une température de réaction comprise entre 80 et 160 °C dans le mélange en réaction, une teneur en propylène de 1 % en poids (sur la base du poids total du mélange réactionnel composé des phases liquide et gazeuse) et une teneur en eau ou en alcool inféreur de 5 mol.-% (par rapport au fluorure d'hydrogène) ne soient pas ateintes et ne soient pas dépassées par une élévation locale de la concentration au point d'introduction, et que la durée de séjour de la phase liquide se situe au-dessous de 20 minutes.

2. Procédé selon la revendication 1, caractérisé en ce que la teneur en eau ou en l'alcool inférieur est maintenue en permanence au-dessous de 2 mol.-%.

3. Procédé selon la revendication 1 ou 2, caractérisé en ce qu'il est exécuté sous une pression totale de 50 à 150 bar.

4. Procédé selon l'une quelconque des revendications 1 à 3, caractérisé en ce qu'il est exécuté à une température comprise entre 100 et 140 °C.

5. Procédé selon l'une quelconque des revendications 1 à 4, caractérisé en ce qu'il est exécuté avec une durée de séjour de la phase liquide de 2 à 10 mn.

6. Procédé selon l'une quelconque des revendications 1 à 5, caractérisé en ce qu'il est exécuté en présence de 5 à 15 mol de HF par mol de propylène.

7. Procédé selon l'une quelconque des revendications 1 à 6, caractérisé en ce qu'on met en réaction du méthanol en tant qu'alcool inférieur.

8. Procédé selon l'une quelconque des revendications 1 à 7, caractérisé en ce que l'eau ou l'alcool inférieur et le propylène sont introduits de façon continue, dans un rapport molaire de (0,9 à 1,1) : 1.

9. Procédé selon l'une quelconque des revendications 1 à 8, caractérisé en ce que l'oxyde de carbone et le propylène sont mélangés à l'état gazeux et sont mis en réaction dans le fluorure d'hydrogène sous la forme de ce mélange.

10. Procédé selon l'une quelconque des revendications 1 à 9, caractérisé en ce qu'il est exécuté dans un réacteur à gaz-liquide à phase gazeuse dispersée.